# DEMANDE DE BREVET EUROPEEN

(11) **EP 4 686 484 A2**
(43) Date de publication de la demande: **04.02.2026**
(21) Numéro de dépôt: 25193089.7
(22) Date de dépôt: 31.07.2025
(51) Int. Cl.: A61L 2/10, A61L 2/24

(54) **ENCEINTE DE DÉSINFECTION ET PROCÉDÉ DE DÉSINFECTION ASSOCIÉ**

(30) Priorité: 31.07.2024 FR 2408458
(71) Demandeur: Germitec, 33000 Bordeaux (FR)
(72) Inventeur: POULON, Fanny, 91440 BURES SUR YVETTE (FR); DALLARD, Jérémy, 33400 TALENCE (FR); BERTHIAS, Maxime, 33750 CADARSAC (FR); HAMDOU, Aimrane, 33800 BORDEAUX (FR)
(74) Mandataire: Lavoix

(57) **Abrégé**

L'invention concerne une enceinte de désinfection (10) d'un instrument médical comprenant :
- un volume de désinfection (24) délimité par au moins une paroi (22A-F),
- un dispositif de génération de rayonnements UV (26) configuré pour générer des rayonnements UV dans le volume de désinfection (24), et
- une unité d'identification (28) positionnée de sorte à lire des informations d'identification d'un instrument médical seulement lorsque l'instrument médical est positionné dans le volume de désinfection (24).

## Description

La présente invention concerne une enceinte de désinfection d'un instrument médical et un procédé de désinfection associé.

Afin d'éviter toutes contaminations, il convient de désinfecter un instrument médical entre deux utilisations.

A cet effet, il est connu de désinfecter des instruments médicaux dans des enceintes de désinfection définissant un volume de désinfection fermé. De telles enceintes comprennent par exemple un dispositif de génération de rayonnements UV (Ultra-Violet) permettant notamment d'effectuer une désinfection des instruments médicaux, et plus particulièrement une désinfection haut-niveau.

Il est connu d'identifier l'instrument médical avant de le placer dans l'enceinte de désinfection, afin d'assurer une traçabilité de la désinfection.

Cependant, une telle identification n'est pas entièrement satisfaisante, en ce qu'il existe des risques d'erreur d'identification. En particulier, il existe un risque que l'instrument médical identifié et celui positionné dans l'enceinte ne soient pas les mêmes. Par exemple, rien n'assure que l'instrument positionné dans l'enceinte soit le même que celui ayant été préalablement identifié.

Le but de l'invention est alors de proposer une enceinte de désinfection assurant une traçabilité améliorée et fiable de la désinfection.

A cet effet, l'invention a pour objet une enceinte de désinfection d'un instrument médical comprenant :
- un volume de désinfection délimité par au moins une paroi,
- un dispositif de génération de rayonnements UV configuré pour générer des rayonnements UV dans le volume de désinfection, et
- une unité d'identification positionnée de sorte à lire des informations d'identification d'un instrument médical seulement lorsque l'instrument médical est positionné dans le volume de désinfection.

Grâce à l'unité d'identification, l'instrument médical est identifié uniquement lorsque celui-ci est positionné dans l'enceinte. Ainsi, l'instrument identifié est de manière certaine le même que celui qui est désinfecté.

Suivant d'autres aspects avantageux de l'invention, l'enceinte de désinfection comprend une ou plusieurs des caractéristiques suivantes, prises isolément ou suivant toutes les combinaisons techniquement possibles :
- L'enceinte de désinfection comprend un support de maintien configuré pour maintenir l'instrument médical à l'intérieur du volume de désinfection.
- L'instrument médical comprend un module d'identification comprenant les informations d'identification de l'instrument médical, l'unité d'identification définissant un volume d'identification, le support de maintien étant configuré pour maintenir l'instrument médical de sorte à positionner le module d'identification fixé à l'instrument médical dans le volume d'identification de l'unité d'identification.
- L'unité d'identification de l'instrument médical comprend un lecteur de radio-identification définissant le volume d'identification.
- L'enceinte de désinfection comprend un processeur configuré pour associer les informations d'identification de l'instrument médical à des informations de caractérisation de la désinfection.
- Les informations de caractérisation de la désinfection comprennent au moins le statut de la désinfection.
- L'enceinte de désinfection comprend une unité d'identification additionnelle de l'utilisateur et/ou du patient disposée à l'extérieur du volume de désinfection pour l'acquisition d'informations d'identification de l'utilisateur de l'instrument médical et/ou d'identification du patient pour lequel l'instrument médical a été utilisé.
- L'enceinte de désinfection comprend en outre une unité d'affichage et/ou d'impression et/ou d'export des informations d'identification de l'instrument médical.

L'invention concerne également un procédé de désinfection d'un instrument médical comprenant les étapes successives suivantes :
a. fourniture d'une enceinte telle que décrite ci-dessus,
b. positionnement de l'instrument médical à l'intérieur du volume de désinfection,
c. lecture des informations d'identification de l'instrument médical dans le volume de désinfection par l'unité d'identification, et
d. lancement d'un cycle de désinfection de l'instrument médical seulement lorsque des informations d'identification ont été lues par l'unité de d'identification.

Avantageusement, le procédé comprend en outre une étape d'association des informations d'identification de l'instrument médical à des informations de caractérisation de la désinfection.

L'invention apparaîtra plus clairement à la lecture de la description qui va suivre, donnée uniquement à titre d'exemple non limitatif, et faite en référence aux dessins dans lesquels :
[Fig. 1] la figure 1 est une vue schématique en perspective d'un exemple d'une enceinte de désinfection selon l'invention ;
[Fig. 2] la figure 2 est une vue schématique de face d'un exemple d'une enceinte de désinfection selon l'invention ;
[Fig. 3] la figure 3 est un organigramme des étapes d'un exemple de procédé de désinfection selon l'invention ; et
[Fig. 4] la figure 4 est une vue schématique d'un exemple d'instrument médical propre à être désinfecté par l'enceinte de désinfection de la figure 1.

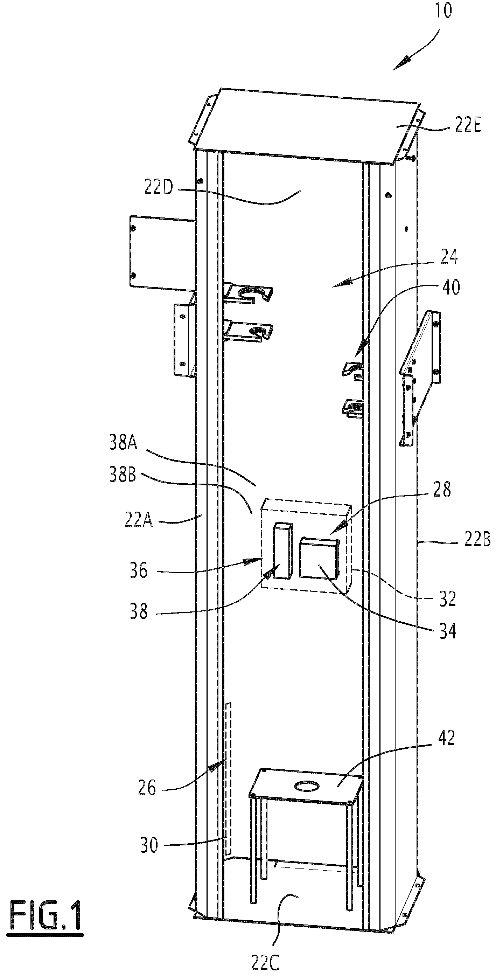

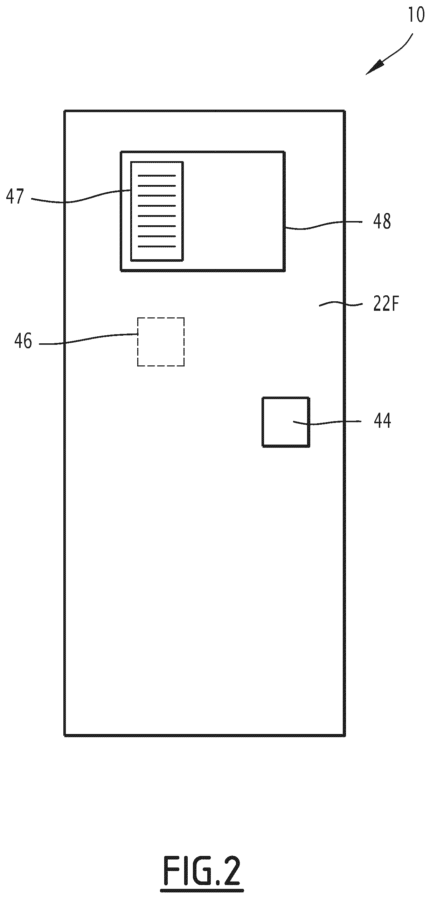

Les figures 1 et 2 illustrent un exemple d'une enceinte de désinfection 10 selon l'invention. L'enceinte de désinfection 10 est configurée pour désinfecter tout ou partie d'un instrument médical 12.

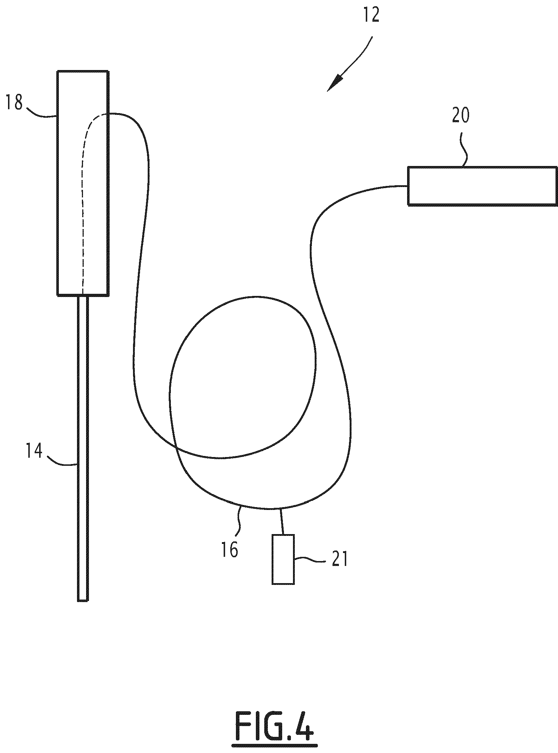

La figure 4 illustre un exemple d'un instrument médical 12 propre à être désinfecté par l'enceinte de désinfection 10.

L'instrument médical 12 comprend par exemple une partie active 14. La partie active 14 est par exemple un tube destiné à être inséré dans le patient, par exemple dans une narine du patient.

De préférence, l'instrument médical 12 comprend une poignée 18 connectée à la partie active 14. La poignée 18 est avantageusement fixée à une extrémité de la partie active 14 et est destinée à être saisie par un utilisateur afin de faciliter l'insertion de la partie active 14 dans le patient.

L'instrument médical 12 comprend, en outre, une partie de raccordement 16 et de préférence également un connecteur 20.

La partie de raccordement 16 est par exemple un câble de raccordement reliant la partie active 14 au connecteur 20.

Le connecteur 20 est par exemple configuré pour être connecté à une unité de mesure et/ou d'affichage, de sorte notamment à collecter et/ou afficher des informations captées par la partie active 14.

L'instrument médical 12 est par exemple une sonde médicale, notamment une sonde médicale échographique, et plus particulièrement une sonde médicale de type « échographie transoephagienne » (ETO), ou une sonde médicale endoscopique, par exemple de type « oto-rhino-laryngologie » (ORL). La sonde médicale est par exemple une sonde pédiatrique ou pour adulte.

Avantageusement, un module d'identification 21 est fixé à l'instrument médical 12.

Par exemple, comme illustré sur la figure 4, le module d'identification 21 est fixé à la partie de raccordement 16 de l'instrument médical 12.

Le module d'identification 21 est par exemple une étiquette.

Le module d'identification 21 comprend de préférence des informations d'identification de l'instrument médical 12.

En particulier, le module d'identification 21 comprend par exemple une puce de radio-identification, également nommée une puce RFID (de l'anglais « radio frequency identification »), contenant ces informations d'identification.

La puce RFID est par exemple une puce à basse fréquence, une puce à haute-fréquence, par exemple du type puce NFC (Near-Field Communication, se traduisant en communication en champ proche), ou une puce UHF (Ultra Haute Fréquence).

En variante, le module d'identification 21 comprend un code-barres.

Ces informations d'identification comprennent par exemple un numéro d'identification de l'instrument médical 12 et/ou le modèle de l'instrument médical 12.

L'enceinte de désinfection 10 comprend un volume de désinfection 24 délimité par au moins une paroi, un dispositif de génération de rayonnements UV 26 et une unité d'identification 28.

Dans un mode de réalisation préférentielle, l'enceinte de désinfection 10 comprend une pluralité de parois 22A, 22B, 22C, 22D, 22E définissant entre elles le volume de désinfection 24.

Plus particulièrement, les parois 22A, 22B, 22C, 22D, 22E, 22F définissent entre elles un volume de désinfection 24 fermé, par exemple de forme parallélépipédique.

Dans le mode de réalisation de l'invention illustré sur les figures 1 et 2, la pluralité de parois comprend par exemple une première paroi latérale 22A et une deuxième paroi latérale 22B opposées l'une de l'autre, un fond 22C et un capot 22E opposé au fond 22C et également une base 22D définissant une troisième paroi latérale et une porte 22F (non représentée sur la figure 1) définissant une quatrième paroi latérale en regard de la base 22D.

En particulier, le capot 22E et la fond 22C sont par exemple perpendiculaires aux deux parois latérales 22A, 22B. De même, la base 22D et la porte 22F sont par exemple perpendiculaires aux deux parois latérales 22A, 22B, au capot 22E et au fond 22C.

Dans une variante non-illustrée, l'enceinte de désinfection 10 comprend une unique paroi 22 délimitant le volume de désinfection 24, qui est par exemple cylindrique ou sphérique.

Le dispositif 26 de génération de rayonnements UV, de préférence UV-C, est configuré pour générer des rayonnements UV dans le volume de désinfection 24.

Par exemple, le dispositif 26 de génération de rayonnements UV comprend une pluralité de sources de rayonnements UV 30 disposées à l'intérieur de l'enceinte de désinfection 10, et par exemple sur au moins l'une des parois 22, par exemple au niveau d'au moins un des bords latéraux de la base 22D comme illustré sur la figure 1.

Chaque source 30 de rayonnements UV-C est par exemple une lampe à mercure générant des rayonnements UV-C. En particulier, chaque lampe à mercure 30 est apte à utiliser du mercure pour exciter un gaz et produire des rayonnements ultraviolets UV-C.

Par rayonnements UV-C, on entend des rayonnements caractérisés par une longueur d'onde comprise entre 200 et 280 nm.

Alternativement, chaque source 30 de rayonnements UV-C est une lampe à LED (Light-Emitting Diode se traduisant en diode électroluminescente).

L'unité d'identification 28 est positionnée de sorte à identifier l'instrument médical 12 seulement lorsque l'instrument médical 12 est positionné dans le volume de désinfection 24.

Par « identifier », on entend plus particulièrement la lecture d'informations d'identification de l'instrument médical 12.

En particulier, lorsque l'instrument médical 12 est positionné en dehors du volume de désinfection 24, l'unité d'identification 28 ne peut pas l'identifier.

Dans le mode de réalisation particulier illustré sur les Figures 1 et 2, l'unité d'identification 28 est positionnée dans le volume de désinfection 24.

Plus particulièrement, dans le mode de réalisation illustré sur la figure 1, l'unité d'identification 28 est fixée à la base 22D de l'enceinte de désinfection 10.

Alternativement, l'unité d'identification 28 est positionnée à l'extérieur du volume de désinfection 24. Par exemple, l'unité d'identification 28 est fixée à une paroi extérieure de l'enceinte de désinfection 10 ou est positionnée à distance de l'enceinte de désinfection 10 par exemple par un bras de maintien.

L'unité d'identification 28 définit par exemple un volume d'identification 32.

Par volume d'identification, on entend le volume à l'intérieur duquel l'unité d'identification 28 est apte à identifier un objet positionné dans ledit volume d'identification 32.

En particulier, un objet positionné en dehors du volume d'identification 32 ne peut pas être identifié par l'unité d'identification 28.

Le volume d'identification 32 est de préférence au moins partiellement compris dans le volume de désinfection 24.

Par exemple, l'unité d'identification 28 comprend un lecteur de radio-identification 34 disposé à l'intérieur du volume de désinfection 24. Le lecteur de radio-identification 34 est configuré pour lire les informations d'identification de l'étiquette d'identification 21 fixée à l'instrument médical 12.

Le lecteur de radio-identification 34 définit de préférence le volume d'identification 32.

Le volume d'identification 32 s'étend par exemple autour du lecteur de radio-identification 34.

Plus précisément, l'unité d'identification 28 est configurée pour identifier l'instrument médical 12 lorsque le module d'identification 21 fixé à l'instrument médical 12 est positionné dans le volume d'indentification 32.

En particulier, dans le cas d'un module d'identification 21 avec une puce RFID basse fréquence destinée à être lue par le lecteur de radio-identification 34, le volume d'identification 32 est par exemple d'environ 10 cm³.

En revanche, dans le cas d'une étiquette d'identification 21 avec une puce UHF destinée à être lue par le lecteur de radio-identification 34, par « à proximité », le volume d'identification 32 est par exemple supérieur à 1 m³.

Avantageusement, l'enceinte de désinfection 10 comprend, en outre, un support de maintien 36 configuré pour maintenir l'instrument médical 12 à l'intérieur du volume de désinfection 24.

De préférence, le support de maintien 36 est configuré pour maintenir l'instrument médical 12 en entier à l'intérieur du volume de désinfection 24.

Autrement dit, lorsque l'instrument médical 12 est maintenu par le support de maintien 36, l'ensemble des éléments constituant l'instrument médical 12 sont disposés à l'intérieur du volume de désinfection 24.

En particulier, la partie active 14, la partie de raccordement 16, la poignée 18 et le connecteur 20 sont entièrement maintenus à l'intérieur du volume de désinfection 24.

Plus particulièrement, les deux extrémités de la partie de raccordement 16 se trouvent à l'intérieur du volume de désinfection 24, lorsque l'instrument médical 12 est maintenu par le support de maintien 36.

Avantageusement, le support de maintien 36 est configuré pour positionner le module d'identification 21 fixé à l'instrument médical 12 à l'intérieur du volume d'identification 32 de l'unité d'identification 28.

A cet effet, comme illustré sur la figure 1, le support de maintien 36 comprend par exemple un premier élément 38 configuré pour positionner le module d'identification 21 fixé à l'instrument médical 12 à l'intérieur du volume d'identification 32 de l'unité d'identification 28.

Plus particulièrement, le premier élément 38 est configuré pour recevoir une portion de la partie de raccordement 16, de sorte à positionner le module d'identification 21 fixé à la partie de raccordement 16 à l'intérieur du volume d'identification 32.

Par exemple, le premier élément 38 est fixé à la base 22D de l'enceinte de désinfection 10.

Avantageusement, le premier élément 38 est configuré pour maintenir une portion de la partie de raccordement 16 de l'instrument médical 12, de préférence la portion de la partie de raccordement 16 portant le module d'identification 21 de sorte à positionner le module d'identification 21 en regard de la surface d'identification 32.

Dans le mode de réalisation particulier illustré sur les figures 1 et 2, le support de maintien 36 comprend une pluralité d'élements secondaires 40, 42. Chaque élément secondaire 40, 42 est de préférence configuré pour maintenir une portion de l'instrument médical 12, comme par exemple la partie active 14, la poignée 18 et/ou le connecteur 20.

Avantageusement, l'enceinte de désinfection 10 comprend, en outre, une unité d'identification additionnelle 44 de l'utilisateur et/ou du patient pour l'acquisition d'informations d'identification de l'utilisateur de l'instrument médical 12 et/ou d'identification du patient pour lequel l'instrument médical 12 a été utilisé.

L'unité d'identification additionnelle 44 est, de préférence, disposée à l'extérieur du volume de désinfection 24. Autrement dit, l'unité d'identification additionnelle 44 ne se trouve pas à l'intérieur de l'enceinte de désinfection 10.

Par exemple, comme illustré sur la figure 2, l'unité d'identification additionnelle 44 est agencée sur la face externe de la porte 22F de l'enceinte de désinfection 10.

Dans un mode de réalisation particulier, l'unité d'identification additionnelle 44 est un lecteur de code-barres, par exemple configuré pour lire un code-barres d'un badge d'un utilisateur utilisant l'instrument médical 12 et/ou le code-barres d'un dossier d'un patient et/ou le code-barres d'un dossier d'un patient pour lequel l'instrument médical 12 a été utilisé antérieurement.

Les informations d'identification de l'utilisateur de l'instrument médical 12 et/ou d'identification du patient comprennent par exemple le nom et le prénom de l'utilisateur, respectivement du patient.

De manière avantageuse, l'enceinte de désinfection 10 comprend, en outre, un processeur 46 propre à mettre en œuvre un cycle de désinfection à l'intérieur du volume de désinfection.

Le processeur 46 est un circuit électronique conçu pour manipuler et/ou transformer des données représentées par des quantités électroniques ou physiques dans des registres du processeur et/ou des mémoires en d'autres données similaires correspondant à des données physiques dans les mémoires de registres ou d'autres types de dispositifs d'affichage, de dispositifs de transmission ou de dispositifs de mémorisation.

En tant qu'exemples spécifiques, le processeur 46 est réalisé sous forme d'un composant logique programmable, tel qu'un FPGA (de l'anglais *Field Programmable Gate Array*), ou encore d'un circuit intégré, tel qu'un ASIC (de l'anglais *Application Specific Integrated Circuit*)*.*

En variante, lorsque le procédé est réalisé sous forme d'un ou plusieurs logiciels, c'est-à-dire sous forme d'un programme d'ordinateur, également appelé produit programme d'ordinateur, il est en outre apte à être enregistré sur un support, non représenté, lisible par ordinateur. Le support lisible par ordinateur est par exemple un medium apte à mémoriser des instructions électroniques et à être couplé à un bus d'un système informatique. A titre d'exemple, le support lisible est un disque optique, un disque magnéto-optique, une mémoire ROM, une mémoire RAM, tout type de mémoire non-volatile (par exemple FLASH ou NVRAM) ou une carte magnétique. Sur le support lisible est alors mémorisé un programme d'ordinateur comprenant des instructions logicielles.

De préférence, le processeur 46 est configuré pour associer les informations d'identification de l'instrument médical 12 à des informations de caractérisation de la désinfection.

Les informations de caractérisation de la désinfection comprennent par exemple le statut de la désinfection.

En particulier, le statut de désinfection comprend un horodatage de la désinfection et/ou l'état de la désinfection, par exemple un « OK » ou un « Succès » si le cycle de désinfection s'est bien déroulé et un « NOK » ou un « Echec » et la raison de l'échec si le cycle de désinfection a échoué.

A cet effet, le processeur 46 est de préférence connecté à l'unité d'identification 28, par exemple par une connexion filaire.

De préférence, le processeur 46 est également configuré de sorte à ne pas déclencher le cycle de désinfection si aucun instrument médical 12 n'a été identifié au préalable dans l'enceinte de désinfection 10 par l'unité d'identification 28.

Par ailleurs, avantageusement, le processeur 46 est configuré également pour associer les informations d'identification de l'instrument médical 12 aux informations d'identification de l'utilisateur de l'instrument médical 12 et/ou d'identification du patient pour lequel l'instrument médical 12 a été utilisé.

A cet effet, le processeur 46 est de préférence connecté à l'unité d'identification additionnelle 44 de l'utilisateur et/ou du patient, par exemple par une connexion filaire.

Avantageusement, les informations d'identification de l'instrument médical 12, et de préférence les informations de caractérisation de la désinfection et avantageusement les informations d'identification de l'utilisateur de l'instrument médical 12 et/ou d'identification du patient associées, sont mémorisées dans une mémoire du processeur 46.

Le processeur 46 est de préférence configuré pour générer un rapport 47 comprenant l'ensemble des informations associées.

En particulier, le rapport 47 comprend au moins les informations d'identification de l'instrument médical 12 et les informations de caractérisation de la désinfection, et de préférence également les informations d'identification de l'utilisateur de l'instrument médical 12 et/ou d'identification du patient pour lequel l'instrument médical 12 a été utilisé.

De manière avantageuse, l'unité de désinfection 10 comprend en outre une unité d'affichage et/ou d'impression et/ou d'export 48 des informations d'identification de l'instrument médical 12.

Plus particulièrement, l'unité d'affichage et/ou d'impression et/ou d'export 48 est configurée pour afficher et/ou imprimer le rapport 47 généré par le processeur 46 ou pour transmettre le rapport 47 généré par le processeur 46 par exemple par un export numérique.

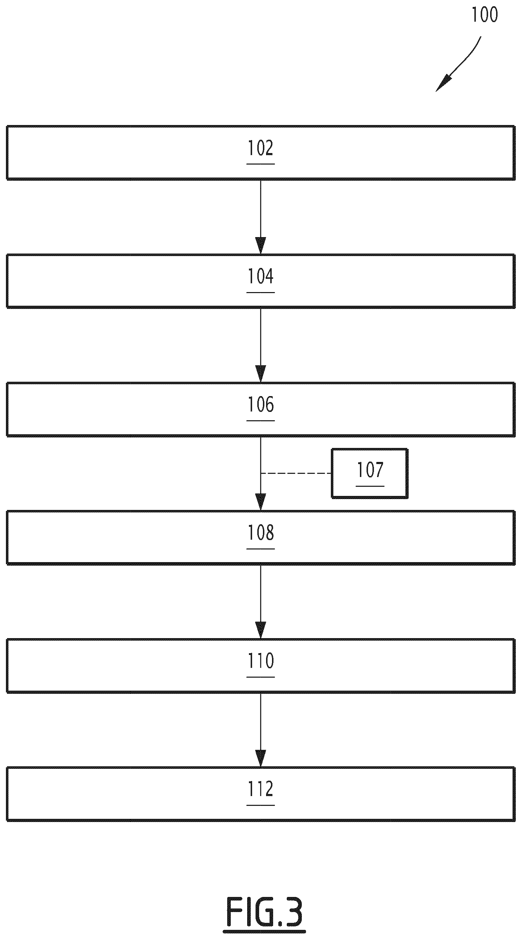

Un procédé de désinfection 100 de l'instrument médical 12 dans l'enceinte de désinfection 10 va maintenant être décrit en référence à la figure 3.

Premièrement, dans une première étape 102, une enceinte de désinfection 10 telle que décrite ci-dessus est fournie. La porte 22F est ouverte, de sorte à donner accès au volume de désinfection 24.

Ensuite, dans une deuxième étape 104, un instrument médical 12 destiné à être désinfecté est positionné dans le volume de désinfection 24 de l'enceinte de désinfection 10.

En particulier, l'instrument médical 12 est positionné en entier à l'intérieur du volume de désinfection 24 et est positionné de sorte à être maintenu par le support de maintien 36.

Plus précisément, dans un mode de réalisation préféré de l'invention, grâce au support de maintien 36, le module d'identification 21 fixé à l'instrument médical 12 est positionnée dans le volume d'identification 32 de l'unité d'identification 28.

Dans une troisième étape 106, l'instrument médical 12 est identifié dans le volume de désinfection 24 par l'unité d'identification 28.

En particulier, l'unité d'identification 28, et plus particulièrement le lecteur de radio-identification 34, lit les informations d'identification de l'instrument médical 12, plus particulièrement contenues dans le module d'identification 21.

Lors de cette troisième étape 106, les informations d'identification de l'instrument médical 12 sont de préférence envoyées au processeur 46.

La porte 22F est ensuite fermée.

Dans un mode de réalisation particulier, le procédé 100 comprend, en outre, une étape 107 d'acquisition d'informations d'identification de l'utilisateur et/ou du patient, en particulier par l'unité d'identification additionnelle 44.

Dans une étape 108, le cycle de désinfection de l'instrument médical 12 est lancé seulement lorsque des informations d'identification ont été lues par l'unité de d'identification 28.

En particulier, par le processeur 46 lance le cycle de désinfection uniquement s'il a reçu des informations d'identification de l'instrument médical 12.

Le cycle de désinfection est adapté pour désinfecter tout ou partie de l'instrument médical 12 positionné dans le volume de désinfection 24.

Plus précisément, le processeur 46 donne par exemple un ordre de commande au dispositif 26 de génération de rayonnements UV. L'ordre de commande comprend par exemple la durée du cycle et la dose de rayonnements UV-C.

Avantageusement, le processeur 46 adapte son ordre de commande au dispositif 26 de génération de rayonnements UV en fonction de l'instrument médical 12 identifié.

De manière avantageuse, le procédé 100 comprend une étape 110 d'association des informations d'identification de l'instrument médical 12 aux informations de caractérisation de la désinfection, et de préférence également aux informations d'identification de l'utilisateur et/ou du patient récoltées à l'étape 108.

De préférence, les informations d'identification de l'instrument médical 12, et avantageusement également les informations lors de cette étape 110, sont mémorisées.

Lors de cette étape 110, le processeur 46 génère par exemple le rapport 47 comprenant l'ensemble des informations associées.

De préférence, le procédé 100 comprend en outre une étape 112 d'impression et/ou d'affichage et/ou d'export de ces informations associées. Par exemple, cette étape 112 comprend l'impression d'un ticket physique et/ou numérique comprenant le rapport 47. De référence, cette étape 112 comprend également la sauvegarde de ces informations.

Une telle enceinte de désinfection 10 est particulièrement avantageuse pour la désinfection d'instruments médicaux 12, et en particulier de sondes médicales.

En particulier, grâce à l'unité d'identification 28 positionnée dans l'enceinte de désinfection 10, l'instrument médical 12 est identifié uniquement lorsqu'il est positionné dans l'enceinte. Ainsi, l'instrument 12 identifié est de manière certaine le même que celui qui est désinfecté.

Par ailleurs, l'association des informations d'identification de l'instrument médical 12 avec les autres informations, notamment celles de caractérisation de la désinfection et éventuellement celles d'identification de l'utilisateur et/ou du patient, permet d'assurer une traçabilité améliorée de l'utilisation et de la désinfection de l'instrument médical 12.

En outre, la génération d'un rapport 47 contenant l'ensemble de ces informations fournit une documentation précise et fiable de l'utilsation et de la désinfection de l'instrument médical 12, ce qui facilite la tracabilité de l'utilsation et de la désinfection de l'instrument médical 12.

L'homme du métier comprendra que les modes de réalisation et variantes précédemment décrits peuvent être combinés entre eux pourvu qu'ils soient compatibles techniquement.

## Revendications

1. Enceinte de désinfection (10) d'un instrument médical (12) comprenant :
- un volume de désinfection (24) délimité par au moins une paroi (22A-F),
- un dispositif de génération de rayonnements UV (26) configuré pour générer des rayonnements UV dans le volume de désinfection (24), et
- une unité d'identification (28) positionnée de sorte à lire des informations d'identification d'un instrument médical (12) seulement lorsque l'instrument médical (12) est positionné dans le volume de désinfection (24).

2. Enceinte de désinfection (10) selon la revendication 1, comprenant un support de maintien (36) configuré pour maintenir l'instrument médical (12) à l'intérieur du volume de désinfection (24).

3. Enceinte de désinfection (10) selon la revendication 2, dans laquelle l'instrument médical (12) comprend un module d'identification (21) comprenant les informations d'identification de l'instrument médical (12), l'unité d'identification (28) définissant un volume d'identification (32), le support de maintien (36) étant configuré pour maintenir l'instrument médical (12) de sorte à positionner le module d'identification (21) fixé à l'instrument médical (12) dans le volume d'identification (32) de l'unité d'identification (28).

4. Enceinte de désinfection (10) selon la revendication 3, dans laquelle l'unité d'identification (28) de l'instrument médical (12) comprend un lecteur de radio-identification (34) définissant le volume d'identification (32).

5. Enceinte de désinfection (10) selon l'une quelconque des revendications 1 à 4, comprenant un processeur (46) configuré pour associer les informations d'identification de l'instrument médical (12) à des informations de caractérisation de la désinfection.

6. Enceinte de désinfection (10) selon la revendication 5, dans laquelle les informations de caractérisation de la désinfection comprennent au moins le statut de la désinfection.

7. Enceinte de désinfection (10) selon l'une quelconque des revendications 1 à 6, comprenant une unité d'identification additionnelle (44) de l'utilisateur et/ou du patient disposée à l'extérieur du volume de désinfection (24) pour l'acquisition d'informations d'identification de l'utilisateur de l'instrument médical et/ou d'identification du patient pour lequel l'instrument médical (12) a été utilisé.

8. Enceinte de désinfection (10) selon l'une quelconque des revendications 1 à 7, comprenant en outre une unité d'affichage et/ou d'impression et/ou d'export (48) des informations d'identification de l'instrument médical.

9. Procédé de désinfection d'un instrument médical (12) comprenant les étapes successives suivantes :
a. fourniture d'une enceinte (10) selon l'une quelconque des revendications précédentes,
b. positionnement de l'instrument médical (12) à l'intérieur du volume de désinfection (24),
c. lecture des informations d'identification de l'instrument médical (12) dans le volume de désinfection (24) par l'unité d'identification (28), et
d. lancement d'un cycle de désinfection de l'instrument médical (12) seulement lorsque des informations d'identification ont été lues par l'unité de d'identification (28).

10. Procédé selon la revendication 9, comprenant en outre une étape d'association des informations d'identification de l'instrument médical (12) à des informations de caractérisation de la désinfection.
